# EUROPEAN PATENT APPLICATION

(11) **EP 4 342 860 A1**
(43) Date of publication of application: **27.03.2024**
(21) Application number: 23197410.6
(22) Date of filing: 14.09.2023
(51) Int. Cl.: C03C 3/089, C03C 3/091, C03C 3/112, C03C 3/115, C03C 4/00, C03C 4/08

(54) **GLASS COMPOSITION AND DENTAL COMPOSITION**

(30) Priority: 21.09.2022 JP 2022150421
(71) Applicant: GC Corporation, Sunto-gun, Shizuoka 410-1307 (JP)
(72) Inventor: FUNAYAMA, Naoya, Tokyo, 174-8585 (JP); MIYAKE, Takahiro, Tokyo, 174-8585 (JP)
(74) Representative: Müller-Boré & Partner Patentanwälte PartG mbB

(57) **Abstract**

A glass composition contains cesium (Cs), at least one of potassium (K) or sodium (Na), and silicon (Si), wherein a content of the cesium (Cs) in terms of oxide is 9% by mass or more, wherein when the potassium (K) is contained in the glass composition, a content of the potassium (K) in terms of oxide is 5% by mass or more, wherein when the sodium (Na) is contained in the glass composition, a content of the sodium (Na) in terms of oxide is 1% by mass or more, and wherein the glass composition does not contain barium (Ea) or contains 0.5% by mass or less of barium (Ba) in terms of oxide.

## Description

### BACKGROUND OF THE INVENTION

### 1. Field of the Invention

The present invention relates to a glass composition and a dental composition.

### 2. Description of the Related Art

In the field of dentistry, glass containing cesium (Cs) is used as a raw material for dental materials fillers that require X-ray contrast because of its high X-ray opacity. For example, Patent Document 1 discloses glass containing Cs₂O as a filler used for dental materials.

### [Related-Art Documents]

### [Patent Documents]

[Patent Document 1] Japanese Laid-open Patent Publication No. 2019-131457

### SUMMARY OF THE INVENTION

With conventional glass, the refractive index tends to increase as the X-ray contrast increases, so it is difficult to achieve both a high X-ray contrast and a low refractive index.

The present invention provides a glass composition that provides a glass that achieves both high X-ray contrast and low refractive index.

The glass composition according to one aspect of the present invention contains cesium (Cs), at least one of potassium (K) or sodium (Na), and silicon (Si), wherein a content of the cesium (Cs) is 9% by mass or more in terms of oxide, wherein when the potassium (K) is contained in the glass composition, a content of the potassium (K) is 5% by mass or more in terms of oxide, wherein when the sodium (Na) is contained in the glass composition, a content of the sodium (Na) is 1% by mass or more in terms of oxide, and barium (Ba) is not contained or a content of the barium (Ba) is 0.5% by mass or less in terms of oxide.

According to one aspect of the present invention, a glass composition that provides a glass that achieves both high X-ray contrast and low refractive index can be achieved.

### DESCRIPTION OF THE PREFERRED EMBODIMENTS

Hereafter, the embodiments of the present invention will be described in detail.

### <Glass Composition>

The glass composition according to the present embodiment contains cesium (Cs), at least one of potassium (K) or sodium (Na), and silicon (Si).

Since cesium (Cs) has an atomic number (number of electrons) of 55, which is close to barium (Ba) with an atomic number (number of electrons) of 56, cesium (Cs) also indicates high X-ray opacity similar to barium (Ba). Furthermore, cesium (Cs) has an ionic radius of 1.67, which is larger than that of barium (Ba) with an ionic radius of 1.35 (the density of electrons in the atom is low or sparse). Therefore, the increase in refractive index can be suppressed compared with barium (Ba).

Cesium (Cs) can be present as an oxide (cesium oxide (Cs₂O)) of cesium (Cs) in glass compositions.

The content of cesium (Cs) in terms of oxide in the glass composition is 9% by mass or more, preferably 12% by mass or more, and more preferably 15% by mass or more. The content of cesium (Cs) in terms of oxide indicates the mass content of cesium oxide (Cs₂O), which is an oxide of cesium (Cs), in the glass composition.

When the content of cesium (Cs) in terms of oxide is 9% by mass or more, the X-ray opacity of the resulting glass can be enhanced, and the X-ray contrast can be enhanced. Although the upper limit of the content of cesium (Cs) in terms of oxide is not particularly limited, the content of cesium (Cs) is preferably 50% by mass or less, more preferably 40% by mass or less, and even more preferably 30% by mass or less, from the viewpoint of suppressing decrease in the chemical durability of the glass composition.

Potassium (K) can be present as an oxide of potassium (K) (potassium oxide (K₂O)) in the glass composition.

When potassium (K) is included, the content of potassium (K) in terms of oxide in the glass composition is 5% by mass or more, preferably 8% by mass or more, and more preferably 10% by mass or more. The content of potassium (K) in terms of oxide indicates the mass content of potassium oxide (K₂O), which is the oxide of potassium (K), in the glass composition.

When potassium (K) is contained in the glass composition, the melting temperature of the glass composition is lowered when the content of potassium (K) in terms of oxide is 5% by mass or more. When potassium (K) is combined with cesium (Cs), the chemical durability of the glass composition can be improved by the mixed alkali effect. Although the upper limit of the content of potassium (K) in terms of oxide is not particularly limited, the upper limit of the content is preferably 20% by mass or less, more preferably 15% by mass or less, and even more preferably 13% by mass or less, from the viewpoint of both suppressing decrease of the chemical durability of the glass composition and easily manufacturing the glass composition.

Sodium (Na) can be present as an oxide of sodium (Na) (sodium oxide (Na₂O)) in the glass composition.

When sodium (Na) is contained in the glass composition, the content of sodium (Na) is 1% by mass or more in terms of oxide, preferably 3% by mass or more, and more preferably 5% by mass or more. The content of sodium (Na) in terms of oxide indicates the mass content of sodium oxide (Na₂O), which is the oxide of sodium (Na), in the glass composition.

When sodium (Na) is contained in the glass composition, if the content of sodium (Na) in terms of oxide is 1% by mass or more, the melting temperature of the glass composition is lowered, easily manufacturing glass composition. When sodium (Na) is combined with cesium (Cs), the chemical durability of the glass composition can be improved by the mixed alkali effect. Although the upper limit of the content of sodium (Na) in terms of oxide is not particularly limited, the upper limit of the content is preferably 25% by mass or less, more preferably 22% by mass or less, and even more preferably 20% by mass or less, from the viewpoint of suppressing decrease in the chemical durability of the glass composition.

Silicon (Si) can be present as an oxide of silicon (Si) (silicon oxide (SiO₂)) in the glass composition.

The content of silicon (Si) in terms of oxide is not particularly limited, but is preferably 20% by mass or more, more preferably 30% by mass or more, and even more preferably 35% by mass or more. The content of silicon (Si) in terms of oxide indicates the mass content of silicon oxide (SiO₂), which is the oxide of silicon (Si), in the glass composition.

When the content of silicon (Si) in terms of oxide is 20% by mass or more, the viscosity of the glass melt can be adjusted and the mechanical strength of the glass composition can be enhanced. Although the upper limit of the content of silicon (Si) in terms of oxide is not particularly limited, the upper limit of the content is preferably 75% by mass or less, more preferably 70% by mass or less, and even more preferably 65% by mass or less, from the viewpoint of reducing the production cost, because the glass melt hardens as the silicon (Si) in the glass composition increases.

The glass composition of the present embodiment does not contain barium (Ba). In this specification, "barium (Ba) free" means substantially free of barium (Ba) except for unavoidable impurities.

When barium (Ba) is contained as an unavoidable impurity, the content of barium (Ba) in terms of oxide is 0.5% by mass or less, preferably 0.3% by mass or less, and more preferably 0.1% by mass or less. When the content of barium (Ba) in terms of oxide exceeds 0.5% by mass, the electron density of the glass increases and the refractive index may increase.

The glass composition of the present embodiment preferably further contains boron (B). Boron (B) can be present as an oxide of boron (B) (boron oxide (B₂O₃) ) in the glass composition.

The content of boron (B) in terms of oxide is not particularly limited, but is preferably 20% by mass or less, more preferably 19% by mass or less, and even more preferably 18% by mass or less. The content of boron (B) in terms of oxide indicates the mass content of boron oxide (B₂O₃), which is an oxide of boron (B), in the glass composition.

When the content of boron (B) in terms of oxide is 20% by mass or less, the melting point of the glass in the glass composition can be lowered to facilitate the production of the glass composition. Although the lower limit of the content of boron (B) in terms of oxide is not particularly limited, the lower limit of the content is preferably 1% by mass or more, more preferably 2% by mass or more, and even more preferably 3% by mass or more, from the viewpoint of both improving the chemical durability of the glass composition and the easily manufacturing the glass composition.

The glass composition of the present embodiment preferably further contains aluminum (Al). Aluminum (Al) can be present as an oxide of aluminum (Al) (aluminum oxide (Al₂O₃)) in the glass composition.

The content of aluminum (Al) in terms of oxide is not particularly limited, but is preferably 15% by mass or less, more preferably 14% by mass or less, and even more preferably 13% by mass or less. The content of aluminum (Al) in terms of oxide indicates the mass content of aluminum oxide (Al₂O₃), which is the oxide of aluminum (Al), in the glass composition.

When the content of aluminum (Al) in terms of oxide is 15% by mass or less, the chemical durability of the glass composition can be improved and the devitrification of the glass can be suppressed. Although the lower limit of the content of aluminum (Al) in terms of oxide is not particularly limited, the lower limit of the content is preferably 0.1% by mass or more, more preferably 0.5% by mass or more, and even more preferably 1% by mass or more, from the viewpoint of both improving the chemical durability of the glass composition and preventing the devitrification of the glass.

The glass composition of the present embodiment further preferably contains fluorine (F). Fluorine (F) can be present as fluoride or fluoride salts in the glass composition.

The content of fluorine (F) is not particularly limited, but is preferably 6% by mass or less, more preferably 5.5% by mass or less, and even more preferably 5% by mass or less. The content of fluorine (F) indicates the mass content of fluorine (F) as a single substance in the glass composition.

When the content of fluorine (F) is 6% by mass or less, the refractive index of the resulting glass can be adjusted. The lower limit of the content of fluorine (F) is not particularly limited, but is preferably 0.1% by mass or more, more preferably 1% by mass or more, and even more preferably 2% by mass or more, from the viewpoint of lowering the viscosity of the glass composition and improving the producibility and workability of the glass composition.

The glass composition of the present embodiment may contain other elements as long as the elements do not detract from the purpose of the invention. Examples of other elements include Sr, Zn, Y, Zr, Ti, and the like from the viewpoint of improving X-ray opacity and chemical durability. Other elements can be present as oxides in the glass composition. The content of other elements is not particularly limited and can be included in the glass composition by any mass.

In the glass composition of the present embodiment, the refractive index (nd) of the glass containing the glass composition is preferably 1.524 or less, more preferably 1.520 or less, even more preferably 1.518 or less, and particularly preferably 1.516 or less. In this specification, the refractive index (nd) refers to the refractive index at the D-line (light rays of wavelength 589 nm).

When the refractive index (nd) of the glass composition of the present embodiment is 1.524 or less, the difference of the refractive index between the glass composition and the dental resin becomes smaller, and the resulting dental composition with high transparency can be obtained. Although the lower limit of the refractive index (nd) is not particularly limited, the range of the refractive index (nd) to achieve a dental composition having high transparency is preferably 1.3 or more, more preferably 1.4 or more, and even more preferably 1.45 or more.

The form of the glass composition is not particularly limited, but may be, for example, powdery (powder), tabular, columnar, filiform, or fibrous. When the form of the glass composition is powder, each component of the glass composition can be easily mixed. When the form of the glass composition is tabular or columnar, the glass composition can be used for optical elements (for example, lenses, prisms, filters, or the like).

The particle size of the powdery glass composition is not particularly limited, but is preferably in a range from 0.01 um to 20 um in median diameter, more preferably in a range from 0.05 um to 10 um, and even more preferably in a range from 0.1 um to 1 um. Here, the particle size means the average particle size defined by the median diameter.

When the particle size of the glass composition is 0.01 um or more, the workability of the glass composition is improved when the glass composition is used as a powder. When the particle size of the glass composition is 20 um or less, the mechanical strength of the dental composition obtained when the glass composition is applied to dental materials is improved.

In the glass composition of the present embodiment, the X-ray contrast of the glass containing the glass composition is 300% or higher, preferably 350% or higher, and more preferably 400% or higher with respect to aluminum. In this specification, X-ray contrast refers to the X-ray contrast measured by the radio-opacity test method of dental materials in accordance with ISO 4049:2019.

In the present embodiment, when the X-ray contrast of the glass containing the glass composition is 300% or higher with respect to aluminum, the glass composition providing high X-ray contrast can be provided.

The use of the glass composition in the present embodiment is not particularly limited, but the glass composition can be used, for example, for dental materials. Dental materials to which the glass composition in the present embodiment can be applied include, for example, dental composite resins, dental cements, dental adhesives, dental temporary sealants, dental primers, dental coating materials, dental hard resins, dental cutting resin materials, dental temporary restorations, dental fillers, toothpastes, and the like.

In addition, the glass composition in the present embodiment can be applied in fields other than dental materials. Applications in fields other than dental materials to which the glass composition of the present embodiment can be applied include, for example, optical elements such as optical lenses, prisms, and optical filters; ultraviolet and infrared cut glass; radiation shielding glass; and the like.

The glass composition of the present embodiment, as described above, contains cesium (Cs), at least one of potassium (K) or sodium (Na), and silicon (Si), the content of cesium (Cs) in terms of oxide is 9% by mass or more, the content of potassium (K) in terms of oxide is 5% by mass or more, the content of sodium (Na) in terms of oxide is 1% by mass or more, and barium (Ba) is not contained or the content of barium (Ba) in terms of oxide is 0.5% by mass or less.

This configuration allows the glass composition of the present embodiment to have high X-ray opacity of the resulting glass, while the electron density is low, so that a glass with both high X-ray contrast and a low refractive index can be obtained. Moreover, since the melting temperature of the glass containing the glass composition of the present invention can be lowered, the glass can be easily produced and processed, and can be applied as a material for various fields. Moreover, since the glass composition of the present embodiment is barium-free as described above, the increase in the electron density of the resulting glass is suppressed, and the refractive index of the glass can be lowered.

Since the glass composition of the present embodiment contains boron (B) and the content of the boron (B) in terms of oxide is 20% by mass or less, the refractive index of the resulting glass can be lowered because the atomic number (number of electrons) of boron (B) is as small as 5, such that the number of electrons is small. In addition, the glass composition of the present embodiment contains 20% by mass or less of boron (B) in terms of oxide, which lowers the melting temperature of the glass, and its composition contributes to the easily manufacturing glass.

As described above, the glass composition of the present embodiment contains aluminum (Al), and when the content of aluminum (Al) in terms of oxide is 15% by mass or less, the refractive index of the resulting glass can be lowered without lowering the X-ray contrast. In addition, the glass composition of the present embodiment has an aluminum (Al) content of 15% by mass or less in terms of oxide, which lowers the melting temperature of the glass, while maintaining the chemical durability of the glass composition without lowering the X-ray contrast of the resulting glass, easily manufacturing glass.

The glass composition of the present embodiment contains fluorine (F), as described above, and when the fluorine (F) content is 6% by mass or less, the refractive index can be further lowered without lowering the X-ray contrast of the resulting glass.

In the glass composition of the present embodiment, when the refractive index (nd) of the glass containing the glass composition is 1.524 or less, the difference of the refractive index between the glass composition and the dental resin becomes smaller, and the dental composition with high transparency and excellent aesthetics can be obtained.

In the glass composition of the present embodiment, when the X-ray contrast of the glass containing the glass composition is 300% or higher when compared with respect to aluminum, the glass with high X-ray opacity and high X-ray contrast can be obtained.

### <Dental Composition>

The dental composition according to the present embodiment includes the glass composition of the present embodiment described above.

Specifically, the glass composition contained in the dental composition according to the present embodiment contains cesium (Cs), at least one of potassium (K) or sodium (Na), and silicon (Si), the content of cesium (Cs) in terms of oxide is 9% by mass or more, the content of potassium (K) in terms of oxide is 5% by mass or more, the content of sodium (Na) in terms of oxide is 1% by mass or more, and the glass composition does not contain barium (Ba) or the content of barium (Ba) in terms of oxide is 0.5% by mass or less.

In the dental composition according to the present embodiment, the effect obtained by the glass composition of the present embodiment can also be obtained in the dental composition in the same manner by including the glass composition of the present embodiment.

That is, since the dental composition of the present embodiment contains the glass with high X-ray opacity and low refractive index, the dental composition with both high X-ray contrast and excellent aesthetics can be obtained. Such an excellent aesthetics results in the close refractive indexes between the glass and resins. Moreover, since the dental composition of the present embodiment exhibits high X-ray contrast, the restoration portion can be clarified in the diagnosis and examination by X-ray image after treatment. Furthermore, since the glass composition contained in the dental composition of the present embodiment is sufficiently adjusted to a suitable refractive index range, the difference of the refractive index between the glass composition and the dental resin become smaller. Therefore, the dental composition of the present embodiment can be suitably used for dental materials requiring aesthetics (for example, dental composite resins, dental cements, dental coats, and the like).

Furthermore, since the glass composition contained in the dental composition of the present embodiment can lower the melting temperature, the production of the dental composition of the present embodiment is easily performed. In addition, in the dental composition of the present embodiment, since the glass composition contained in the dental composition is barium-free, the increase in the electron density of the glass contained in the glass composition is suppressed and the refractive index can be lowered.

### EXAMPLES

The present invention will be described below with further examples. In the following, the unitless value or "%" is the mass standard (% by mass) unless otherwise noted.

### <Preparation of Glass>

Glass (Examples 1 to 14, Comparative Example 1) was prepared with the compositions indicated in Table 1. A batch of raw materials obtained by mixing various raw materials was placed in a platinum crucible, kept in an electric furnace preheated at 1400°C to 1500°C for 30 to 60 minutes, and poured out into a carbon mold to obtain a bulk material. Glass was obtained as a glass powder with a median diameter (average particle size) of 0.4 um by grinding. A refractive index of the resulting glass powder (glass) was measured. The bulk material of the resulting glass was annealed the below procedure; the bulk-glass was heated from room temperature to 600°C at a rate of 20°C/min in an electric furnace, and was kept at 600°C for 30 minutes, then slowly cooled to room temperature. The bulk material of the glass after heat treatment was sliced, and slice pieces were also obtained. The X-ray contrast (with respect to Al%) was measured for the resulting glass slices. The refractive index and X-ray contrast (with respect to Al%) of the glass are indicated in Table 1.

**[Table 1]**

| % by mass | Examples | | | | | | | | | | | | | | Comparative Example |
|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 1 |
| SiO₂ | 67.0 | 64.5 | 57.9 | 57.4 | 62.5 | 46.5 | 61.0 | 57.4 | 56.6 | 53.5 | 45.8 | 40.4 | 43.6 | 44.0 | 79.5 |
| Na₂O | 17.3 | 16.6 | 14.9 | 7.4 | 8.1 | 6.0 | 7.9 | | | | 7.9 | 7.0 | 1.1 | 7.6 | 20.5 |
| K₂O | | | | | | | | 11.2 | 11.1 | 11.2 | | | 5.0 | | |
| Cs₂O | 15.7 | 18.9 | 27.2 | 26.9 | 29.4 | 40.8 | 28.7 | 26.9 | 26.6 | 26.7 | 28.6 | 25.4 | 27.3 | 27.5 | |
| B₂O₃ | | | | 8.3 | | 6.7 | | | | 4.1 | 17.7 | 15.7 | 16.9 | 8.5 | |
| Al₂O₃ | | | | | | | | | 1.2 | | | 11.5 | 6.1 | 12.4 | |
| F | | | | | | | 2.4 | 4.5 | 4.5 | 4.5 | | | | | |
| Total | 100 | 100 | 100 | 100 | 100 | 100 | 100 | 100 | 100 | 100 | 100 | 100 | 100 | 100 | 100 |
| Refractive index | 1.495 | 1.500 | 1.515 | 1.510 | 1.505 | 1.520 | 1.490 | 1.488 | 1.488 | 1.495 | 1.515 | 1.508 | 1.500 | 1.524 | 1.487 |
| X-ray imaging (with respect to Al%) | 314 | 406 | 619 | 619 | 749 | 939 | 618 | 642 | 587 | 639 | 606 | 499 | 639 | 638 | 67 |

### <Refractive Index>

Liquid with a specific refractive index and the prepared glass powder were mixed in a ratio of 1:1 (mass ratio) and packed in a transparent tube. The transparent tube was then held over a white LED to visually observe the color of the transmitted light in the transparent tube. The refractive index of the liquid, which makes the transmitted light of the transparent tube green in color, was then adopted as the refractive index of the glass powder. When the refractive index of the liquid is lower than that of the glass powder, the color of the transmitted light of the transparent tube is blue. When the refractive index of the liquid is higher than that of the glass powder, the color of light transmitted through the transparent tube is red or yellow.

When the refractive index of the glass powder is in a range from 1.4600 to 1.5400, Liquid 1, Liquid 2, or a mixture of these liquids listed in Table 2 were used as the liquids. Both Liquid 1 and Liquid 2 are common dental materials.

**[Table 2]**

| | | | | | | | |
|---|---|---|---|---|---|---|---|
| Refractive index [-] | 1.4600 | 1.4604 | 1.4608 | 1.4612 | 1.4616 | ··· | 1.5400 |
| Liquid 1 [% by mass] | 1000 | 99.5 | 99.0 | 98.5 | 98.0 | ··· | 0.0 |
| Liquid 2 [% by mass] | 0.0 | 0.5 | 1.0 | 1.5 | 2.0 | ··· | 1000 |

| | | | | | | | |
|---|---|---|---|---|---|---|---|
| Liquid 1: Triethylene glycol dimethacrylate Liquid 2: Ethoxylated bisphenol A dimethacrylate | | | | | | | |

### <X-ray contrast >

X-ray contrast of the glass composition is measured by a radio-opacity test method for dental materials in accordance with ISO 4049:2019. Specifically, an optical density or gray value of a specimen (tabular glass composition) with a thickness Ts (unit: mm) is measured. Next, an aluminum plate with a thickness of Ta (unit: mm) having the same optical density or gray value of the specimen of thickness Ts is determined. The percentage of Ta to Ts (unit: %) is then used as the X-ray contrast value.

From Table 1, the glass composition containing 9% by mass or more of cesium oxide (Cs₂O), 5% by mass or more of potassium oxide (K₂O), 1% by mass or more of sodium oxide (Na₂O), and barium (Ba)-free demonstrated a refractive index (nd) of 1.524 or less in the resulting glass, and an X-ray contrast of the resulting glass with respect to that of aluminum was 300% or higher (Examples 1 to 14).

In contrast, the glass composition that does not contain cesium oxide (Cs₂O) and potassium oxide (K₂O) demonstrated an X-ray contrast of the resulting glass with respect to that of aluminum that was less than 300% (Comparative Example 1).

Embodiments disclosed above include, for example, the following aspects.

### (Appendix 1)

A glass composition containing:
cesium (Cs); at least one of potassium (K) or sodium (Na); and
silicon (Si),
wherein a content of the cesium (Cs) in terms of oxide is 9% by mass or more,
wherein when the potassium (K) is contained in the glass composition, a content of the potassium (K) in terms of oxide is 5% by mass or more,
wherein when the sodium (Na) is contained in the glass composition, a content of the sodium (Na) in terms of oxide is 1% by mass or more, and
wherein the glass composition does not contain barium (Ba) or contains 0.5% by mass or less of barium (Ba) in terms of oxide.

### (Appendix 2)

The glass composition according to Appendix 1 further comprising boron (B), wherein a content of the boron (B) in terms of oxide is 20% by mass or less.

### (Appendix 3)

The glass composition according to Appendix 1 or 2 further comprising aluminum (Al), wherein a content of the aluminum (Al) in terms of oxide is 15% by mass or less.

### (Appendix 4)

The glass composition according to any one of Appendices 1 to 3 further comprising fluorine (F), wherein a content of the fluorine (F) is 6% by mass or less.

### (Appendix 5)

The glass composition according to any one of Appendices 1 to 4, wherein a refractive index (nd) of a glass containing the glass composition is 1.524 or less.

### (Appendices 6)

The glass composition according to any one of Appendices 1 to 5, wherein an X-ray contrast of the glass containing the glass composition is 300% or higher with respect to an X-ray contrast of aluminum.

### (Appendices 7)

A dental composition contains the glass composition of any one of Appendices 1 to 6.

As described above, the embodiment of the present invention is not limited to a specific embodiment, and various modifications and changes are possible within the scope of the invention described in the claims.

This patent application is based on and claims priority to Japanese Patent Application No. 2022-150421 filed on September 21, 2022, the entire contents of which are incorporated herein by reference.

## Claims

1. A glass composition comprising:
cesium (Cs);
at least one of potassium (K) or sodium (Na); and
silicon (Si),
wherein a content of the cesium (Cs) in terms of oxide is 9% by mass or more,
wherein when the potassium (K) is contained in the glass composition, a content of the potassium (K) in terms of oxide is 5% by mass or more,
wherein when the sodium (Na) is contained in the glass composition, a content of the sodium (Na) in terms of oxide is 1% by mass or more, and
wherein the glass composition does not contain barium (Ba) or contains 0.5% by mass or less of barium (Ba) in terms of oxide.

2. The glass composition according to claim 1 further comprising boron (B), wherein a content of the boron (B) in terms of oxide is 20% by mass or less.

3. The glass composition according to claim 1 or 2 further comprising aluminum (Al), wherein a content of the aluminum (Al) in terms of oxide is 15% by mass or less.

4. The glass composition according to any one of claims 1 to 3 further comprising fluorine (F), wherein a content of the fluorine (F) is 6% by mass or less.

5. The glass composition according to any one of claims 1 to 4, wherein a refractive index (nd) of a glass containing the glass composition is 1.524 or less.

6. The glass composition according to any one of claims 1 to 5, wherein an X-ray contrast of a glass containing the glass composition is 300% or higher with respect to an X-ray contrast of aluminum.

7. A dental composition comprising the glass composition of any one of claims 1 to 6.
